(19) Europäisches Patentamt
European Patent Office
Office européen des brevets

(11) Publication number: 0 189 300 B1

(12) # EUROPEAN PATENT SPECIFICATION

(45) Date of publication of patent specification: 06.03.91

(51) Int. Cl.5: C07D 249/10, C07D 405/04, A01N 43/653

(21) Application number: 86300373.7

(22) Date of filing: 20.01.86

The file contains technical information submitted after the application was filed and not included in this specification

(54) Dihydrotriazole derivatives and their use as herbicides.

(30) Priority: 23.01.85 JP 10623/85
15.03.85 JP 51700/85

(43) Date of publication of application:
30.07.86 Bulletin 86/31

(45) Publication of the grant of the patent:
06.03.91 Bulletin 91/10

(84) Designated Contracting States:
DE FR GB

(56) References cited:
EP-A- 0 070 089
FR-A- 2 526 271
JP-A-57 193 406
JP-A-57 193 466
JP-A-58 185 572

JOURNAL OF PHARMACEUTICAL SCIENCES, vol. 59, no. 8, August 1970, pages 1190-1191, Easton, US; P.K. KADABA: "Triazolines VI: Evaluation of 1,5-diaryl-delta2-1,2,3-triazolines and arylidene anilines for herbicidal activity"

(73) Proprietor: KUREHA KAGAKU KOGYO KABUSHIKI KAISHA
9-11 Horidome-cho 1-chome Nihonbashi
Chuo-ku
Tokyo 103(JP)

(72) Inventor: Shida, Takafumi
1-6 Ochiai Nishiki-machi
Iwaki-shi Fukushima-ken(JP)
Inventor: Watanabe, Takeo
78-31 Hananoi Nishiki-machi
Iwaki-shi Fukushima-ken(JP)
Inventor: Yamazaki, Shiro
59-2 Kaneyama Joban-Nishigomachi
Iwaki-shi Fukushima-ken(JP)
Inventor: Shinkawa, Hiroyasu
14 Suka Nishiki-machi
Iwaki-shi Fukushima-ken(JP)
Inventor: Satake, Keigo
4-8-15 Nakaoka-machi
Iwaki-shi Fukushima-ken(JP)

(74) Representative: Perry, Robert Edward et al
GILL JENNINGS & EVERY 53-64 Chancery Lane
London WC2A 1HN(GB)

EP 0 189 300 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to dihydrotriazole derivatives and their use as herbicides.

Rice, wheat and corn are important crops. It is essential to protect these crops from weeds. A selective herbicide, i.e. which kills the weeds but which does not severely injure the crop, is very desirable.

Kadaba, J. Pharm. Sci. 59 (1970) 1190-1191, discloses 1,5-diaryl-4,5-dihydro-1,2,3-triazoles which were screened for herbicidal activity. FR-A-2526271, EP-A-0070089, JP-A-58185572 JP-A-57193406 and JP-A-57193466 disclose 3-carbamyl-1,5-diaryl-1,2,4-triazoles and their herbicidal activity.

Novel compounds according to the present invention have the formula

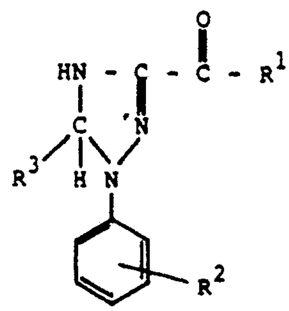

(I)

wherein $R^1$ is amino or $C_{1-4}$ alkoxy; $R^2$ is H, halogen or $C_{1-4}$ alkyl; and $R^3$ is furyl, or phenyl optionally substituted by halogen, hydroxyl, carboxyl, $C_{1-4}$ alkyl or $C_{1-4}$ alkoxy.

According to a second aspect of the present invention, a process for producing a novel compound of the invention comprises reacting an amine of the formula

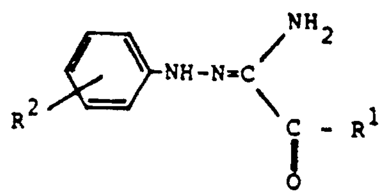

(II)

wherein $R^1$ and $R^2$ are as defined above, with an aldehyde of the formula

$$R^3 - CHO \qquad (III)$$

wherein $R^3$ is as defined above, in the presence of an acid catalyst.

According to a third aspect of the present invention, a herbicidal composition comprises, as an active ingredient, a novel compound of the invention and a diluent therefor.

According to a fourth aspect of the present invention, a method for killing weeds comprises applying to weeds or to the locus thereof a novel compound of the invention, or a composition of the invention.

The novel compounds of the present invention exhibit herbicidal activity against weeds of true grasses (Gramineae) and, especially, broad-leaved weeds. They do not exhibit any severe effect on important crops such as rice, wheat and corn, and therefore they can be safely applied to rice pads and fields. Since they have a suitable soil transport property, they can also be applied effectively to orchards and flower plantations.

In the synthetic process of the invention, the amine (II) is reacted with the aldehyde (III), with dehydration, at a temperature of -20 to 300° C, preferably 0 to 200° C, in the presence of an acid catalyst, and preferably in an inert solvent. The product should not be dehydrogenated and, in order to prevent this side-reaction and maximise the yield of product, the solvent should be deoxygenated in advance and the reaction should proceed under an inert atmosphere. However, if the product is insoluble in the reaction solvent, and almost all the product crystallises during the reaction, the reaction can be carried out under an inert atmosphere.

The acid catalyst may be, for example, an organic acid such as p-toluenesulphonic acid or benzenesulphonic acid, or an inorganic acid such as sulphuric acid or phosphoric acid. However, if an acidic solvent which has acid catalytic activity, such as formic acid, acetic acid, propionic acid or a mixture thereof, is used alone or diluted with a small amount of water or another solvent, a separate acid catalyst is not needed.

An inert solvent, i.e. which does not react with the amine or the aldehyde, can be used. Examples of such solvents are aromatic liquids such as benzene, toluene, xylene, monochlorobenzene or dichlorobenzene, ester-type solvents such as ethyl acetate, chlorinated solvents such as carbon tetrachloride, chloroform or dichloroethane, and acidic solvents as described above. If azeotropic distillation is used to cause dehydration, a water-immiscible solvent should be used. If an acidic solvent as described above is used, the reaction can go at room temperature.

It may be preferred that $R^1$ is amino Compounds of the invention in which $R^1$ is alkoxy are generally less effective as herbicides.

One group of preferred compounds of the invention comprises those of formula I wherein $R^1$ is amino and $R^3$ is phenyl optionally substituted by halogen, hydroxyl, alkyl or alkoxy; more preferably, either $R^2$ is 3-methyl and $R^3$ is phenyl, 4-chlorophenyl, 4-methoxyphenyl or pentafluorophenyl or $R^2$ is 4-chloro and $R^3$ is phenyl, 4-chlorophenyl or 4-methoxyphenyl. Another preferred group comprises those compounds of formula I wherein $R^1$ is amino, $R^2$, is H and $R^3$ is phenyl, 3-methylphenyl, 4-chlorophenyl, 2-hydroxyphenyl, 3-hydroxyphenyl, 4-hydroxyphenyl, 2-carboxyphenyl or furyl.

17 compounds of the invention have been prepared, and they, numbers for their identification, and their melting points are given in Table 1.

Table 1

| Compound No. | Atom or Group | | | Melting point (°C) |
|---|---|---|---|---|
| | $R^1$ | $R^2$ | $R^3$ | |
| 1 | $NH_2$ | H | 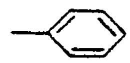 | 208 - 211 |
| 2 | $NH_2$ | H | 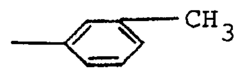 | 194 - 197 |
| 3 | $NH_2$ | H | 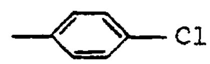 | 213 - 215 |
| 4 | $NH_2$ | H | 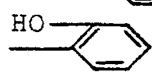 | 182 - 184 |
| 5 | $NH_2$ | H | 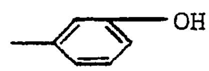 | 174 - 176 |
| 6 | $NH_2$ | H | 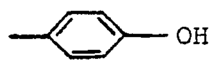 | 210 - 213 |
| 7 | $NH_2$ | H | 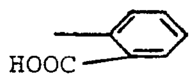 | 145 - 147 |
| 8 | $NH_2$ | 3-$CH_3$ | 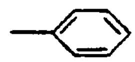 | 193 - 195 |
| 9 | $NH_2$ | 3-$CH_3$ | 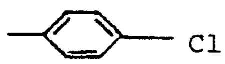 | 195 - 198 |
| 10 | $NH_2$ | 3-$CH_3$ | 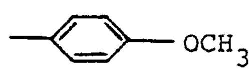 | 176 - 179 |
| 11 | $NH_2$ | 3-$CH_3$ | 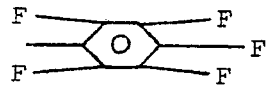 | ca. 140 (decomposed) |
| 12 | $NH_2$ | 4-Cl | 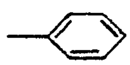 | 199 - 201 |
| 13 | $NH_2$ | 4-Cl | 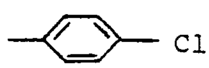 | 121 - 122 |
| 14 | $NH_2$ | 4-Cl | 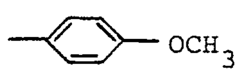 | 176 - 178 |
| 15 | $NH_2$ | H | 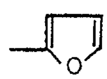 | 183 - 186 |
| 16 | $OCH_3$ | H | 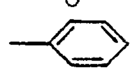 | 148 - 149 |
| 17 | $OCH_3$ | H | 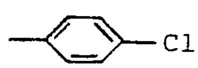 | 143 - 145 |

The following Examples 1 and 2 illustrate how compounds of the invention may be prepared.

Example 1 5-(4-Chlorophenyl)-4,5-dihydro-1-phenyl-1H-1,2,4-triazole-3-carboxamide (compound No. 3)

1.8 g of a compound of formula II ($R^1$ = $NH_2$, $R^2$ = H) and 2.1 g p-chlorobenzaldehyde were introduced into 50 ml benzene together with 20 mg p-toluenesulphonic acid, and refluxed for 10 hours. Water was separated as the benzene azeotrope. After cooling, 3.0 g of the product were separated by filtration (94% yield) : mp 213-215 C; infrared spectrum (KBr, $cm^{-1}$) $\nu$NH-3380, 3260, 3200 $\nu$CO 1660; NMR spectrum ($d_6$-DMSO) $\delta$ (ppm) 6.40 (1H, S: CH<) 6.6 - 8.1 (12H, m; ArH + NH + $NH_2$).

Example 2 4,5-Dihydro-1-(3-methylphenyl)-5-phenyl-1H-1,2,4-triazole-3-carboxamide (compound No. 8)

3.0 g of a compound of formula II ($R^1$ = $NH_2$, $R^2$ = 3-$CH_3$) and 2.1 g benzaldehyde were introduced into 50 ml deoxygenated benzene together with 25 mg p-toluenesulphonic acid and refluxed for 3 hours under a nitrogen atmosphere. After cooling, 4.1 g of crystals of the product were separated by filtration (94% yield): mp 193-195 C; infra-red spectrum (KBr, $cm^{-1}$) $\nu$NH 3380, 3290, 3170 $\nu$CO 1660; NMR spectrum ($d_6$-DMSO) $\delta$ (ppm) 2.23 (2H, S; $CH_3$) 6.50 (1H, S; CH<) 6.6 - 8.4 (13H, m; ArH + NH + $NH_2$); mass spectrum (m/z, relative intensity) 280 ($M^+$, 55%) 279 (20%), 278 (15%), 262 (14%), 203 (100%), 186 (78%).

The same product was obtained when 19.2 g of the same formula II compound and 11.7 g benzaldehyde were mixed and stirred for 1.5 hours at room temperature under a nitrogen atmosphere. Crystalline product was separated by filtration and washed with 20 ml of deoxygenated acetic acid. The washed product was dried in a KOH desiccator. 22.5 g of the product (80.5% yield) were obtained.

The following Examples 3 and 4 illustrate compositions of the invention. All parts are by weight.

Example 3

50 parts compound No. 1, 5 parts lignin sulphonate, 3 parts alkyl sulphonate and 42 parts diatomite were pulverised to give a wettable powder which can be diluted with water to any desired concentration.

Example 4

8 parts compound No. 6, 40 parts bentonite, 45 parts clay and 7 parts lignin sulphonate were uniformly mixed. The mixture was kneaded with water, processed into granules by an extruding granulator, and dried.

The following Examples 5 to 7 illustrate the herbicidal utility of the invention.

Example 5

In tests to determine pre-emergence effect, seeds of various plants (9 weeds and 4 crops) were sown on soil packed in a planter (650 x 210 x 200 mm) and covered with a thin layer of soil. A dilute solution of a compound of formula I (concentration adjusted to correspond to 50 g/are) was uniformly sprayed on to the surface of the soil. The planter was then maintained in a greenhcuse.

After 25 days, the state of the plants was observed, to assess the damage due to the application of the solution, and thus to evaluate both herbicidal activity against the weeds and damage to the crops. Table 2 gives the results, using the following values:

| Herbicidal activity | | Damage | |
|---|---|---|---|
| 0: | No effect | - : | None |
| 1: | 20% effective | ± : | Minute |
| 2: | 40% effective | + : | Slight |
| 3: | 60% effective | ++ : | Medium |
| 4: | 80% effective | +++ : | Severe |
| 5: | 100% effective | ++++ : | Dead |

Example 6

In tests to evaluate post-emergence herbicidal effect, the same range of seeds as in Example 5 was sown on soil packed in a planter (650 x 210 x 200 mm), covered with a thin layer of soil, and maintained in a greenhouse. When the crop or weed reached the two to three leaf stage, an aqueous suspension of a

compound of formula I (concentration adjusted to correspond to 50 g/are) was uniformly sprayed on to the surface of the foliage and the soil. After 25 days further maintenance in the greenhouse, herbicidal activity and crop damage were evaluated as in Example 5; the results are recorded in Table 3.

Example 7

In tests to determine herbicidal efficacy against rice-pad weeds by soil treatment before rice plantation, three pots, each having a plantable area of 1/5000 are, were packed with soil and adjusted to a rice-pad-like state. The first pot was sown with Echinochloa crus-galli in two stages, so that one group of the weed plants was at the three-leaf stage and another group was before germination, at the same time of treatment. The second pot was sown with Cyperus microiria in two stages, so that one group of the weed plants was at the two-leaf stage and another group was before germination, at the time of treatment. The third pot had no weed seeds.

An aqueous suspension of a compound of formula I was applied to the water layer of each pot, in an amount corresponding to 30 g/are. Three days after treatment, the third pot was planted with rice plant seedlings. All the pots were maintained in a greenhouse. 25 days after treatment, herbicidal activity and crop damage were evaluated as in Example 5; the results are recorded in Table 4.

Table 2

| Plant | Number of present compound | | | | | | | | | | | | Not treated |
|---|---|---|---|---|---|---|---|---|---|---|---|---|---|
| | 1 | 2 | 3 | 4 | 5 | 6 | 8 | 9 | 10 | 12 | 13 | 14 | |
| Weeds | Herbicidal activity against weeds | | | | | | | | | | | | |
| Echinochloa crus-galli | 4 | 5 | 4 | 5 | 5 | 5 | 5 | 5 | 5 | 4 | 4 | 4 | 0 |
| Cyperus iria | 5 | 5 | 4 | 5 | 5 | 5 | 5 | 5 | 5 | 5 | 4 | 4 | 0 |
| Poa annua | 5 | 5 | 4 | 4 | 4 | 4 | 4 | 5 | 5 | 4 | 5 | 5 | 0 |
| Stallaria media | 5 | 5 | 5 | 5 | 5 | 5 | 5 | 5 | 5 | 5 | 4 | 4 | 0 |
| Cardamine flexuosa | 5 | 5 | 5 | 5 | 5 | 5 | 5 | 5 | 5 | 5 | 5 | 5 | 0 |
| Bidens frondosa | 5 | 5 | 4 | 5 | 5 | 5 | 5 | 5 | 5 | 5 | 5 | 5 | 0 |
| Portulaca oleracea | 5 | 5 | 4 | 4 | 4 | 5 | 5 | 5 | 5 | 5 | 5 | 4 | 0 |
| Euxolus ascendens | 5 | 5 | 5 | 5 | 5 | 5 | 5 | 5 | 5 | 5 | 5 | 5 | 0 |
| Persicaria logiseta | 5 | 5 | 5 | 5 | 5 | 5 | 5 | 5 | 5 | 4 | 5 | 5 | 0 |
| Crops | Damage to crops | | | | | | | | | | | | |
| Corn | - | - | - | - | - | - | - | - | - | - | - | - | - |
| Soy-bean plant | + | - | - | - | - | - | - | + | + | - | - | - | - |
| Cotton plant | - | - | - | - | - | - | - | - | - | - | - | - | - |
| Wheat | - | - | - | - | - | - | - | - | - | - | - | - | - |

Table 3

| Plant | Number of present compound | | | | | | | | | | | | Not treated |
|---|---|---|---|---|---|---|---|---|---|---|---|---|---|
| | 1 | 2 | 3 | 4 | 5 | 6 | 8 | 9 | 10 | 12 | 13 | 14 | |
| Weeds | Herbicidal activity against weeds | | | | | | | | | | | | |
| Echinochloa crus-galli | 3 | 5 | 3 | 4 | 5 | 5 | 5 | 4 | 4 | 3 | 4 | 3 | 0 |
| Cyperus iria | 4 | 5 | 4 | 5 | 5 | 5 | 4 | 4 | 5 | 4 | 3 | 3 | 0 |
| Poa annua | 4 | 4 | 4 | 5 | 4 | 4 | 3 | 5 | 4 | 4 | 5 | 4 | 0 |
| Stallaria media | 5 | 5 | 5 | 5 | 5 | 5 | 5 | 5 | 5 | 5 | 5 | 5 | 0 |
| Cardamine flexuosa | 5 | 5 | 5 | 5 | 5 | 5 | 5 | 5 | 5 | 5 | 5 | 5 | 0 |
| Bidens frondosa | 5 | 5 | 5 | 5 | 5 | 5 | 5 | 5 | 5 | 5 | 5 | 5 | 0 |
| Portulaca oleracea | 5 | 5 | 5 | 5 | 4 | 5 | 4 | 5 | 5 | 4 | 5 | 4 | 0 |
| Euxolus ascendens | 5 | 5 | 5 | 5 | 5 | 5 | 5 | 5 | 5 | 5 | 5 | 5 | 0 |
| Persicaria logiseta | 5 | 5 | 5 | 5 | 5 | 5 | 5 | 5 | 4 | 4 | 5 | 4 | 0 |
| Crops | Damage to crops | | | | | | | | | | | | |
| Corn | - | - | - | - | - | - | - | - | - | - | - | - | - |
| Soy-bean plant | + | - | - | - | + | - | - | + | + | + | + | - | - |
| Cotton plant | - | - | - | - | - | - | - | - | - | - | - | - | - |
| Wheat | - | - | - | - | - | - | - | - | - | - | - | - | - |

Table 4

| Plant | Number of present compound | | | | | | | | | | | | Not treated |
|---|---|---|---|---|---|---|---|---|---|---|---|---|---|
| | 1 | 2 | 3 | 4 | 5 | 6 | 8 | 9 | 10 | 12 | 13 | 14 | |
| | Herbicidal activity against weeds | | | | | | | | | | | | |
| Weeds | | | | | | | | | | | | | |
| Echinochloa crus-galli | | | | | | | | | | | | | |
| 3 leaf stage | 4 | 5 | 4 | 5 | 5 | 5 | 5 | 5 | 4 | 4 | 3 | 4 | 0 |
| pre-germination | 5 | 5 | 5 | 5 | 5 | 5 | 5 | 5 | 5 | 5 | 5 | 5 | 0 |
| Cyperus microiria | | | | | | | | | | | | | |
| 2 leaf stage | 4 | 5 | 3 | 5 | 4 | 4 | 5 | 4 | 5 | 4 | 4 | 4 | 0 |
| pre-germination | 5 | 5 | 4 | 5 | 5 | 5 | 5 | 5 | 5 | 5 | 4 | 5 | 0 |
| | Damage to crops | | | | | | | | | | | | |
| Crops | | | | | | | | | | | | | |
| rice plant | - | - | - | - | - | - | - | - | - | - | - | - | - |

## Claims

1. A compound of the formula

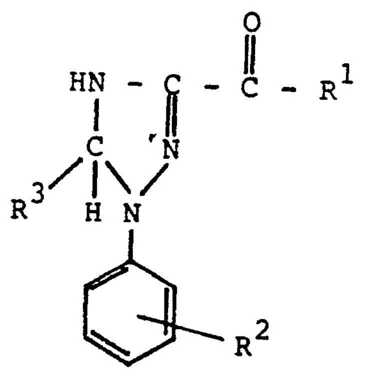

(I)

wherein $R^1$ is amino or $C_{1-4}$ alkoxy; $R^2$ is H, halogen or $C_{1-4}$ alkyl; and $R^3$ is furyl, or phenyl optionally substituted by halogen, hydroxyl, carboxyl, $C_{1-4}$ alkyl or $C_{1-4}$ alkoxy.

2. A compound according to claim 1, wherein $R^1$ is amino.

3. A compound according to claim 2, wherein $R^3$ is phenyl optionally substituted by halogen, hydroxyl, $C_{1-4}$ alkyl or $C_{1-4}$ alkoxy.

4. A compound according to claim 3, wherein $R^2$ is 3-methyl and $R^3$ is phenyl, 4-chlorophenyl, 4-methoxyphenyl or 2,3,4,5,6-pentafluorophenyl.

5. A compound according to claim 3, wherein $R^2$ is 4-chloro and $R^3$ is phenyl, 4-chlorophenyl or 4-methoxyphenyl.

6. A compound according to claim 4, wherein $R^3$ is phenyl.

7. A compound according to claim 2, wherein $R^2$ is H, and $R^3$ is phenyl, 3-methylphenyl, 4-chlorophenyl, 2-hydroxyphenyl, 3-hydroxyphenyl, 4-hydroxyphenyl, 2-carboxyphenyl or furyl.

8. A compound according to claim 7, wherein $R^3$ is 4-chlorophenyl.

9. A compound according to claim 1, wherein $R^1$ is methoxy, R is H and $R^3$ is phenyl or 4-chlorophenyl.

10. A process for producing a compound according to any preceding claim, which comprises reacting an amine of the formula

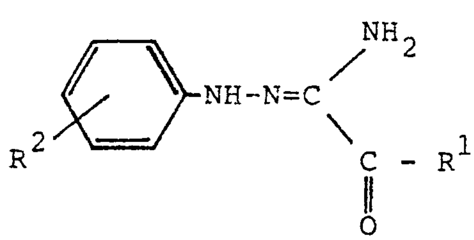

(II)

wherein $R^1$ and $R^2$ are as defined in the preceding claim, with an aldehyde of the formula

$$R^3 - CHO \qquad (III)$$

wherein $R^3$ is as defined in the preceding claim, in the presence of an acid catalyst.

**11.** A herbicidal composition which comprises, as an active ingredient, a compound according to any of claims 1 to 9 and a diluent therefor.

**12.** A method for killing weeds, which comprises applying to weeds or to the locus thereof a compound according to any of claims 1 to 9 or a composition according to claim 11.

## Revendications

**1.** Composé de formule

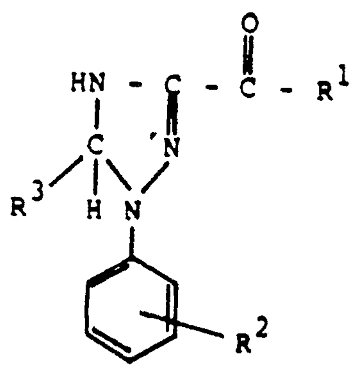

(I)

dans laquelle $R^1$ est un groupe amino ou alcoxy en $C_1$-4; $R^2$ est un atome d'hydrogène ou d'halogène ou un groupe alkyle en $C_{1-4}$; et $R^3$ est le groupe furyle ou un groupe phényle éventuellement substitué par un halogène ou par un groupe hydroxy, carboxy, alkyle en $C_{1-4}$ ou alcoxy en $C_{1-4}$.

**2.** Composé selon la revendication 1, dans lequel $R^1$ est le groupe amino.

**3.** Composé selon la revendication 2, dans lequel $R^3$ est un groupe phényle éventuellement substitué par un halogène ou par un groupe hydroxy, alkyle en $C_{1-4}$ ou alcoxy en

**4.** Composé selon la revendication 3, dans lequel $R^2$ est le groupe 3-méthyle et $R^3$ est le groupe phényle, 4-chlorophényle 4-méthoxyphényle ou 2,3,4,5,6-pentafluorophényle.

**5.** Composé selon la revendication 3, dans lequel $R^2$ est 4-chloro et $R^3$ est le groupe phényle, 4-chlorophényle ou 4-méthoxyphényle.

**6.** Composé selon la revendication 4, dans lequel $R^3$ est le groupe phényle.

**7.** Composé selon la revendication 2, dans lequel $R^2$ est H, et $R^3$ est le groupe phényle, 3-méthylphényle, 4-chlorophényle, 2-hydroxyphényle, 3-hydroxyphényle, 4-hydroxyphényle, 2-carboxyphényle ou furyle.

**8.** Composé selon la revendication 7, dans lequel $R^3$ est le groupe 4-chlorophényle.

**9.** Composé selon la revendication 1, dans lequel $R^1$ est le groupe méthoxy, $R^2$ et H et $R^3$ est le groupe phényle ou 4-chlorophényle.

**10.** Procédé pour la préparation d'un composé selon l'une quelconque des revendications précédentes, lequel comprend la mise en réaction d'une amine de formule

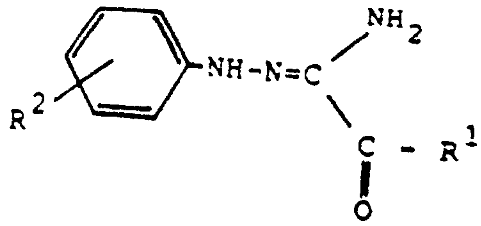

(II)

dans laquelle $R^1$ et $R^2$ sont tels que définis dans la revendication précédente, avec un aldéhyde de formule

$$R^3\text{-CHO} \qquad (III)$$

dans laquelle $R^3$ est tel que défini dans la revendication précédente, en présence d'un catalyseur acide.

11. Composition herbicide, laquelle comprend, en tant que composant actif, un composé selon l'une quelconque des revendications 1 à 9 et un diluant pour celui-ci;

12. Procédé pour la destruction de mauvaises herbes, lequel comprend l'application sur les mauvaises herbes, ou sur le lieu où elles se trouvent, d'un composé selon l'une quelconque des revendications 1 à 9 ou d'une composition selon la revendication 11.

## Ansprüche

1. Verbindung der Formel (I)

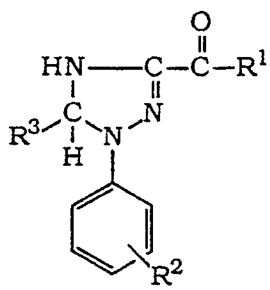

(I)

in der $R^1$ eine Amino- oder $C_{1-4}$-Alkoxygruppe; $R^2$ ein Wasserstoffatom, ein Halogenatom oder eine $C_{1-4}$-Alkylgruppe; und $R^3$ eine Furylgruppe oder Phenylgruppe, die gegebenenfalls durch Halogenatome, Hydroxylgruppen, Carboxylgruppen, $C_{1-4}$-Alkylgruppen oder $C_{1-4}$-Alkoxygruppen substituiert ist.

2. Verbindung nach Anspruch 1, worin $R^1$ eine Aminogruppe darstellt.

3. Verbindung nach Anspruch 2, worin $R^3$ eine gegebenenfalls durch Halogenatome, Hydroxylgruppen, $C_{1-4}$-Alkylgruppen oder $C_{1-4}$-Alkoxygruppen substituierte Phenylgruppe bedeutet.

4. Verbindung nach Anspruch 3, worin $R^2$ eine 3-Methylgruppe und $R^3$ eine Phenylgruppe, eine 4-Chlorphenylgruppe, eine 4-Methoxyphenylgruppe oder eine 2,3,4,5,6-Pentafluorphenylgruppe bedeuten.

5. Verbinbindung nach Anspruch 3, worin $R^2$ ein 4-Chloratom und $R^3$ eine Phenylgruppe, eine 4-Chlorphenylgruppe oder eine 4-Methoxyphenylgruppe bedeuten.

6. Verbinbindung nach Anspruch 4, worin $R^3$ eine Phenylgruppe darstellt.

7. Verbindung nach Anspruch 2, worin $R^2$ ein Wasserstoffatom und $R^3$ eine Phenylgruppe, eine 3-Methylphenylgruppe, eine 4-Chlorphenylgruppe, eine 2-Hydroxyphenylgruppe, eine 3-Hydroxyphenylgruppe, eine 4-Hydroxyphenylgruppe, eine 2-Carboxyphenylgruppe oder eine Furylgruppe darstellen.

**8.** Verbindung nach Anspruch 7, worin $R^3$ eine 4-Chlorphenylgruppe bedeutet.

**9.** Verbindung nach Anspruch 1, worin $R^1$ eine Methoxygruppe, eine R2 ein Wasserstoffatom und $R^3$ eine Phenylgruppe oder eine 4-Chlorphenylgruppe bedeuten.

**10.** Verfahren zur Herstellung einer Verbindung nach einem der vorhergehenden Ansprüche, welches darin besteht, ein Amin der Formel (II)

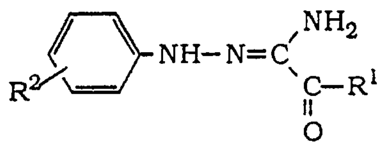

(II)

worin $R^1$ und $R^2$ die in den vorhergehenden Ansprüchen angegebenen Bedeutungen besitzen, mit einem Aldehyd der Formel (III)

$$R^3\text{-}CHO \qquad (III)$$

worin $R^3$ die in den vorhergehenden Ansprüchen angegebene Bedeutung besitzt, in Gegenwart eines Säurekatalysators umsetzt.

**11.** Herbizides Mittel enthaltend als Wirkstoff eine Verbindung nach einem der Ansprüche 1 bis 9 und ein dafür geeignetes Verdünnungsmittel.

**12.** Verfahren zum Abtöten von Unkräutern, welches darin besteht, eine Verbindung nach einem der Ansprüche 1 bis 9 oder ein Mittel nach Anspruch 11 auf die Unkräuter oder ihren Wachstumsbereich aufzubringen.